# EUROPEAN PATENT SPECIFICATION

(11) **EP 4 346 540 B1**
(45) Date of publication and mention of the grant of the patent: **29.04.2026**
(21) Application number: 22728648.1
(22) Date of filing: 25.05.2022
(51) Int. Cl.: A61B 1/00, A61B 1/05, A61B 1/06, A61B 1/07

(54) **AN ENDOSCOPE**
EIN ENDOSKOP
UN ENDOSCOPE

(30) Priority: 28.05.2021 EP 21176573; 28.05.2021 EP 21176578
(43) Date of publication of application: 10.04.2024
(73) Proprietor: Ambu A/S, 2750 Ballerup (DK)
(72) Inventor: SØRENSEN, Morten, 2750 Ballerup (DK)
(74) Representative: AWA Denmark A/S
(86) International application number: PCT/EP2022/064283
(87) International publication number: WO 2022/248592

(56) References cited:
- EP-A1- 3 539 449
- EP-A1- 3 613 326
- US-A1- 2021 068 634

## Description

### Technical Field

The present disclosure relates to insertion endoscopes, in particular but not limited to a tip part of such an endoscope.

### Background

Insertable vision devices such as endoscopes are well known for visually inspecting inaccessible places such as body cavities, e.g. human body cavities. Typically, the endoscope comprises an elongated insertion cord with a handle at the proximal end as seen from the operator and visual inspections means, such as a builtin camera, at the distal end of the elongated insertion cord. Electrical wiring for the electronic vision receptor, e.g. the chip of a camera and other electronics such as LED lighting accommodated in the tip part at the distal end run along the inside of the elongated insertion cord from the handle to the tip part. Instead of using cameras, endoscopes may also be fibre-optic, in which case the optical fibres run along inside of the elongated insertion cord to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion cord from the tip part through the remainder of articulated segments to a control mechanism of the handle. Furthermore, a working channel may run along the inside of the insertion cord from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with at least one camera or similar image capturing device serving as an electronic vision receptor at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above-mentioned LED lighting using one or more Light Emitting Diodes (LEDs) in conjunction with lightguides in the tip of the endoscope, as e.g. formed integrally with a front window covering also the vision receptor as mentioned in EP 3539450 disclosing a disposable endoscope.

The light distribution in the emitted light from a light source such as an optical fibre or an LED is, however, not optimal as regards the field of vision of a vision receptor such as an image sensor, a video camera or an optical fibre. In particular, an LED may spread the emitted light over a wide angle from what is ideally a point source. The vision receptor on the other hand is typically a rectangular array or matrix, with a certain width-height aspect ratio. It may therefore be problematic to have the corners of the field of vision of the vision receptor proper illuminated without overexposure of other parts. As an example, in many body cavities, in particular tubular ones, objects in the periphery of the field of vision will often be closer than those in the centre. Consequently, they will be stronger illuminated than those in the centre, which in turn leads to overexposure of the image at the periphery and underexposure in the centre where the object of interest is often likely to be. The opposite, however, may also be the case so that the centre is overexposed while corners of the field of vision is underexposed.

JP-8-122633 deals with the light distribution to the corners of the field of view of a reusable endoscope. However, rather than using lightguides in front of LEDs JP-8-122633 uses relatively complicated lens systems in front of optical fibres, providing the light at the tip of the endoscope. Prior art document EP 3539449 discloses an endoscope according to the preamble of independent claim 1.

### Summary

Based on this prior art, it is the object of the present disclosure to provide a tip part of an endoscope which overcomes at least some of the above problems.

According to the invention as defined in independent claim 1 this object is achieved by an endoscope comprising a proximal handle and a distal tip with at least one transparent window part, said distal tip being arranged at the distal end of an insertion cord extending from said proximal handle, said distal tip comprising an electronic image capture device having an essentially rectangular receptor defining a vertical imaging direction (V) with a height (h) and a horizontal imaging direction (H) with a width (w), the distal tip comprises at least one light source, a lightguide with a centre axis, where the lightguide comprises a proximal end and a distal end and a circumferential surface extending between said proximal end and said distal end, wherein said circumferential surface is configured to provide internal reflection of light emitted from the light source, and said circumferential surface defines a cross-section of said lightguide, wherein said cross-section defines at least four corners arranged with an angular spacing around said centre axis, and where between two neighbouring corners said circumferential surface has a predetermined curvature between, wherein said predetermined curvature is so selected that incident light emitted from said centre axis is reflected at an angle Θ with respect to said horizontal direction (H), where the angle Θ is either ± arctan (h/w), or ±(180° - arctan (h/w)).

The dependent claims 2 - 14 define preferred embodiments of the claimed endoscope. Claim 15 defines a visualization system comprising a video processing apparatus and an endoscope according to any of claims 1 - 14.

According to an embodiment of the first aspect of the disclosure said curvature is given by the formula: Θ= 2Θ₂ - Θ₁, where Θ₁ is the emission angle of a light ray with respect to said horizontal direction and Θ₂ is the angle of the tangent to the circumferential surface with respect to horizontal at the point of incidence of said light ray. With this curvature rays emanating from the LED at the centre will be reflected in the preferred direction and rays emanating from parts of the LED slightly off-set from the centre axis will still largely be reflected in the desired direction.

According to a further embodiment of the first aspect of the disclosure said cross-section defines four corners arranged with a 90° spacing around said centre axis so as to provide a first pair and a second pair of mutually opposing corners, where a first corner of the first pair opposes the second corner of the first pair along a first diagonal coincident with said vertical imaging direction (V), and where a first corner of the second pair opposes the second corner of the second pair along a second diagonal coincident with said horizontal (H) imaging direction.

According to an embodiment of the first aspect of the disclosure the corners of said first pair of mutually opposing corners and said second pair of mutually opposing corners are concave corners as seen from the inside of the lightguide. This improves the light distribution over embodiments with versions with concave corners only.

According to an embodiment according to the first aspect of the disclosure the number of further corners is a multiple of four, in particular four or eight. Adding further corners in multiples of four, i.e. corresponding multiples of the number of corners in the imaging device may further improve the light distribution of light to those corners.

According to an embodiment of the first aspect of the disclosure, the corners of said first pair of mutually opposing corners and said second pair of mutually opposing corners are convex corners as seen from the inside of the lightguide.

According to a further embodiment according to the first aspect of the disclosure said convex corners have an angle in the interval from 215° to 235°, preferably approximately 225°. This preferred angle, corresponding to 180° + 2 x 22.5° with a proper interval around it, reflects at a 45° angle seen from the centre for the lightguide from where the light ideally enters the lightguide, although the LED is not an ideal point source.

According to an embodiment according to the first aspect of the disclosure, further corners are provided between said first pair of mutually opposing corners and said second pair of mutually opposing corners. This further improves the light distribution towards the corners of the field of view.

According to a further embodiment according to the first aspect of the disclosure said concave corners have an angle in the interval from 125° to 145°, preferably approximately 135°.

According to another embodiment of the first aspect of the invention the side surfaces between said edges are smooth, i.e. without any discontinuities such as concave corners between the convex corners forming the edges. This further improves the control of light emission through and from the lightguide and facilitates manufacturing of the lightguide as it is easier to e.g. injection mould.

According to a further embodiment of the first aspect of the disclosure the distal end face of the of the lightguide constitutes the front window or part of the front window. This may further improve the control of the light emitted from the endoscope directly from the lightguide rather than through a front windowpane or similar object without lightguide properties irrespective of the front windowpane is integral with the lightguide or not.

An endoscope according to the disclosure, wherein the light guide and the window is a single-piece integrally moulded component. This makes the manufacturing cost-efficient.

According to another embodiment of the disclosure, the light guide and the window is a single-piece integrally moulded component. This makes it easier to manufacture and, according to yet a yet further preferred embodiment of the first aspect of the disclosure, allows the angle of the edges with respect to the centre axis to be between 0° and 3°, preferably approximately 1°. This allows for easy manufacture of the lightguide by e.g. injection moulding without inhibiting the desired optical properties.

According to yet another preferred embodiment of the first aspect of the disclosure the maximum angle of the side surfaces with respect to the centre axis is in the interval from 2° to 10°, preferably approximately 5°. This provides a proper curvature to reflect some of the light incident on the side surfaces more towards the corners as seen in the cross-sectional plane and hence emanate in a corresponding direction from the lightguide into the field of view of the image capture device.

According to another embodiment of the disclosure, wherein the light guide and the window is a single-piece integrally moulded component, the single-piece integrally moulded component allows the distal end face of the lightguide to constitute the window part. Thus, the light emitted in the direction of the corners of the field of view will not be disturbed by passing and exiting from a thickness of transparent material. This in turn allows for the omission of any additional window in front of the imaging device except for e.g. lenses integrated in a camera.

According to an embodiment of a second aspect of the disclosure, the object is achieved by a visualization system comprising a video processing apparatus (VPA) and an endoscope according to any of claims 1 - 14. The VPA is configured to receive live video from the endoscope and output a video based on the received live video.

In a variation of the present embodiment, the VPA comprises a display device.

In another variation of the present embodiment, the VPA does not comprise a display device, and the display device is communicatively connected to the VPA, either wirelessly or via a cable.

### Brief Description of the Drawings

The disclosure will now be made in greater detail based on non-limiting exemplary embodiments and with reference to the drawings, on which:
Fig. 1 shows an isometric view of the tip part of an endoscope implementing the disclosure,
Fig. 2 shows the inside of the tip part of Fig. 1 seen from the proximal end,
Figs. 3a-3c shows a first embodiment of a lightguide that may be implemented in the tip part of Fig. 1,
Figs. 3d-3f show emission diagrams for the first embodiment,

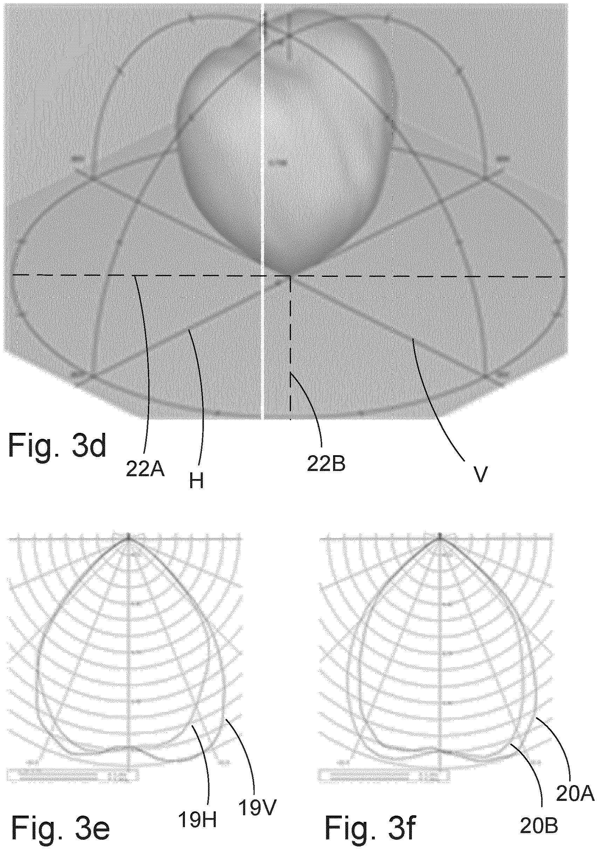

Fig. 3d illustrates luminous intensity distribution for the first embodiment in a 3-dimensional plot,
Fig. 3e illustrates luminous intensity distribution in the horizontal and the vertical plane for the first embodiment,
Fig. 3f illustrates luminous intensity distribution in the two diagonal planes for the first embodiment,

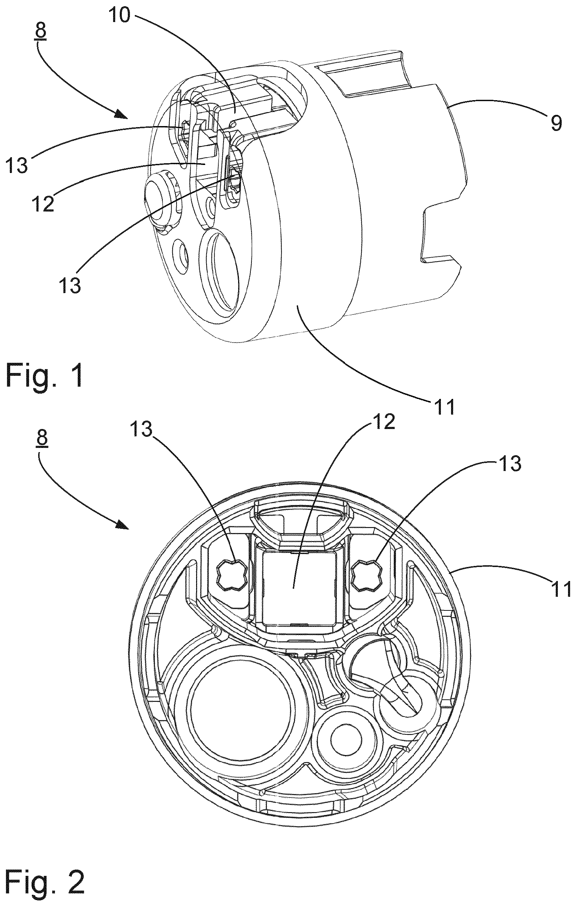

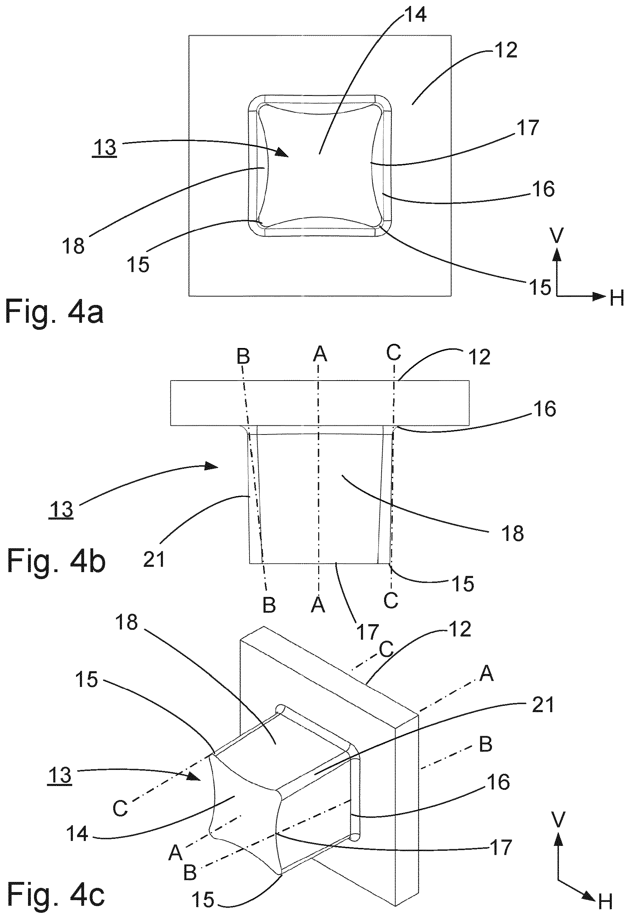

Figs. 4a-4c shows a second embodiment of a lightguide that may be implemented in the tip part of Fig. 1,

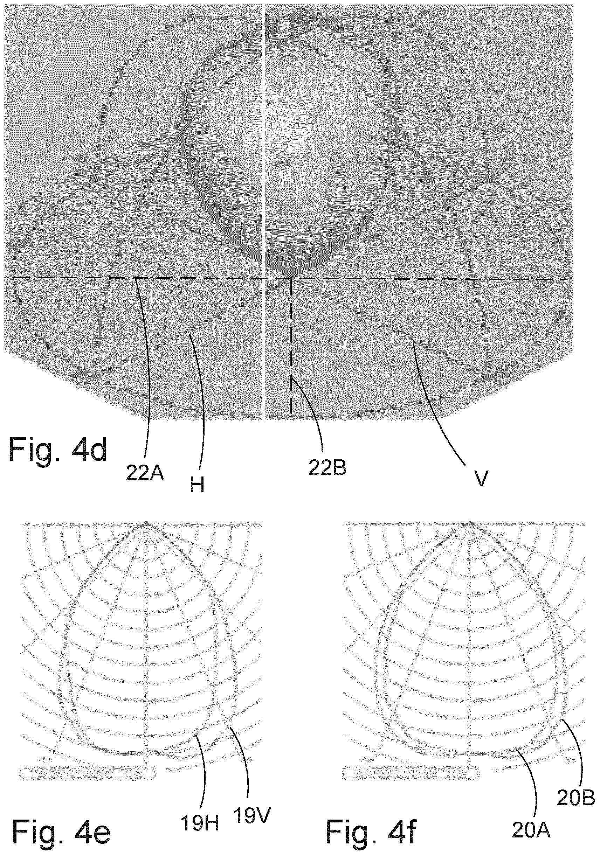

Fig. 4d illustrates luminous intensity distribution for the second embodiment in a 3-dimensional plot,
Fig. 4e illustrates luminous intensity distribution in the horizontal and the vertical plane for the second embodiment,
Fig. 4f illustrates luminous intensity distribution in the two diagonal planes for the second embodiment,

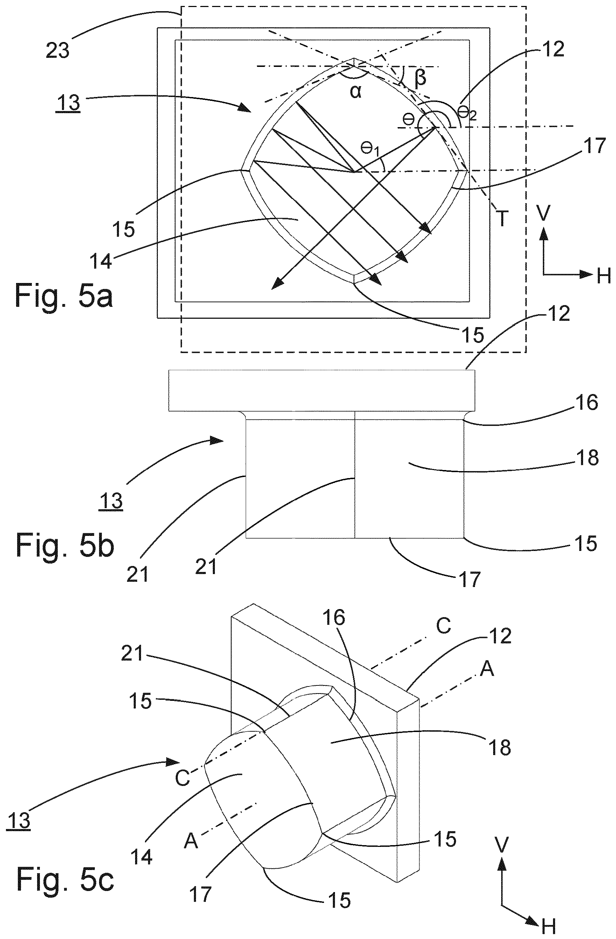

Figs. 5a-5c shows a third embodiment of a lightguide that may be implemented in the tip part of Fig. 1,

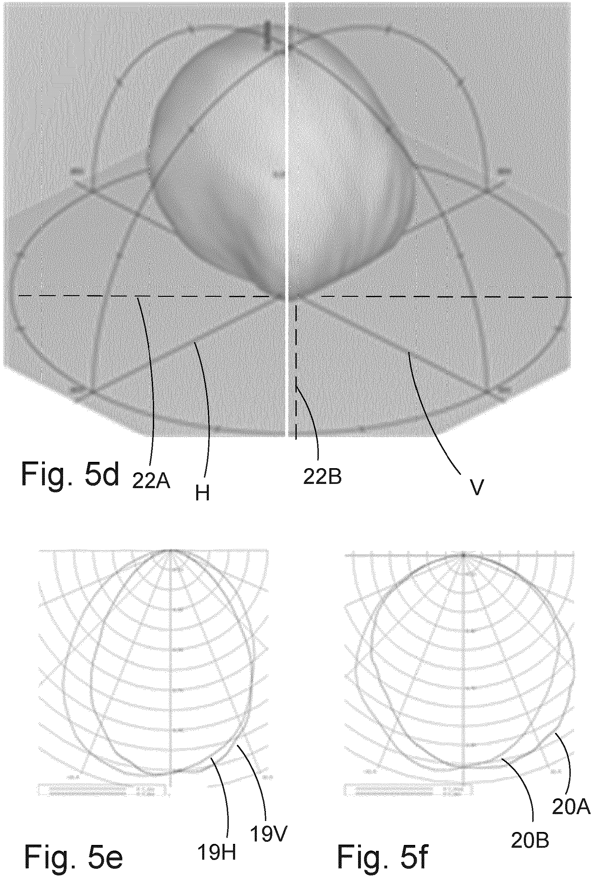

Fig. 5d illustrates luminous intensity distribution for the third embodiment in a 3-dimensional plot,
Fig. 5e illustrates luminous intensity distribution in the horizontal and the vertical plane for the third embodiment,
Fig. 5f illustrates luminous intensity distribution in the two diagonal planes for the third embodiment,

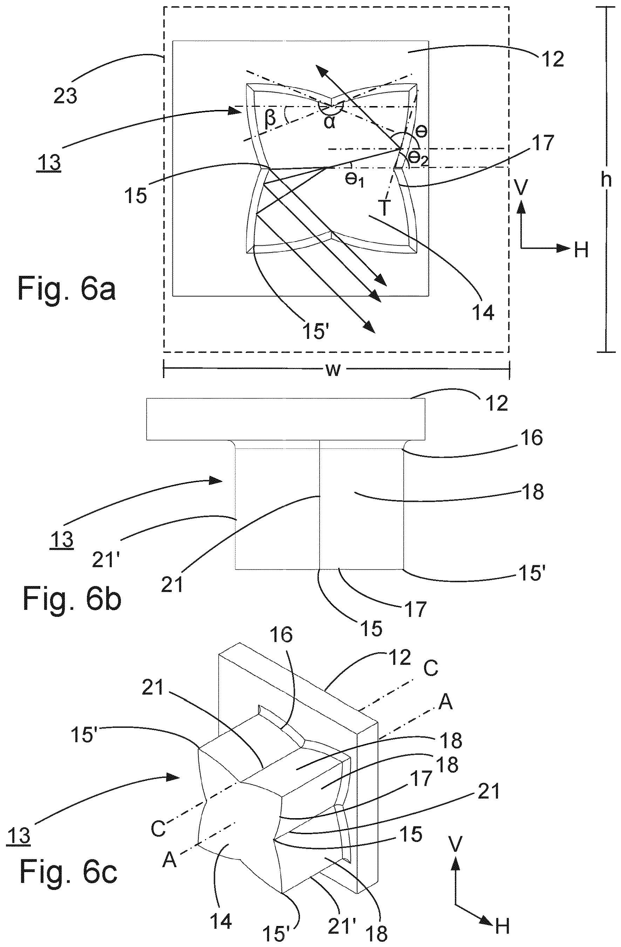

Figs. 6a-6c shows a fourth embodiment of a lightguide that may be implemented in the tip part of Fig. 1,

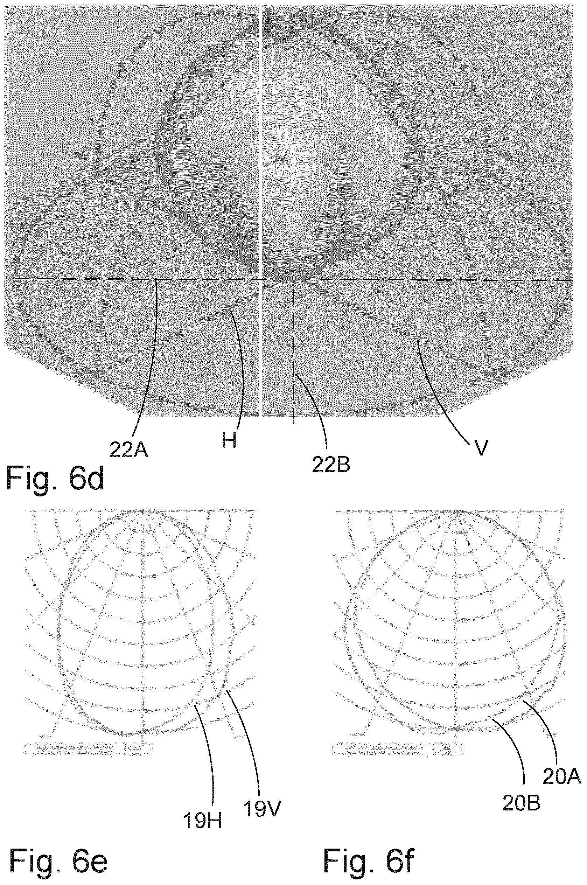

Fig. 6d illustrates luminous intensity distribution for the fourth embodiment in a 3-dimensional plot,
Fig. 6e illustrates luminous intensity distribution in the horizontal and the vertical plane for the fourth embodiment,
Fig. 6f illustrates luminous intensity distribution in the two diagonal planes for the fourth embodiment,

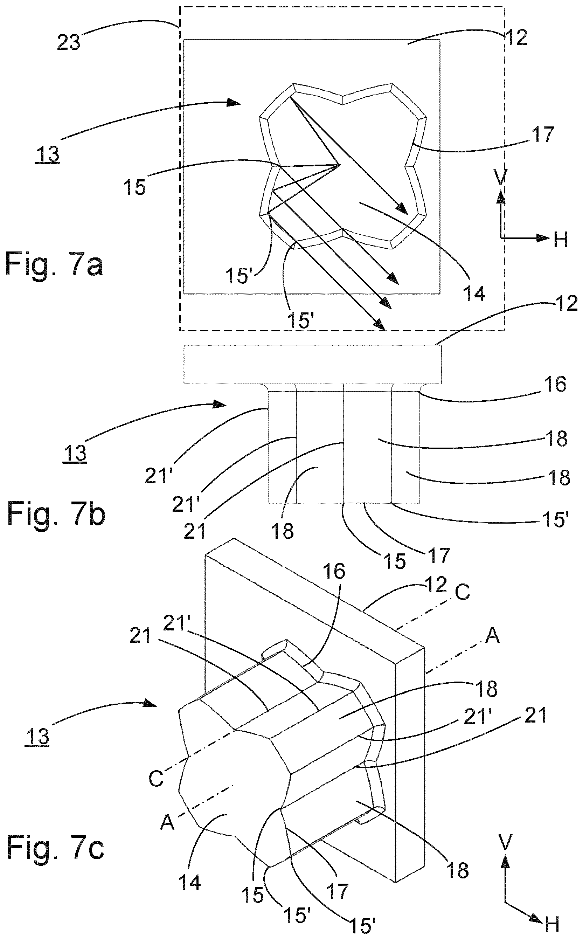

Figs. 7a-7c shows a fifth embodiment of a lightguide that may be implemented in the tip part of Fig. 1,

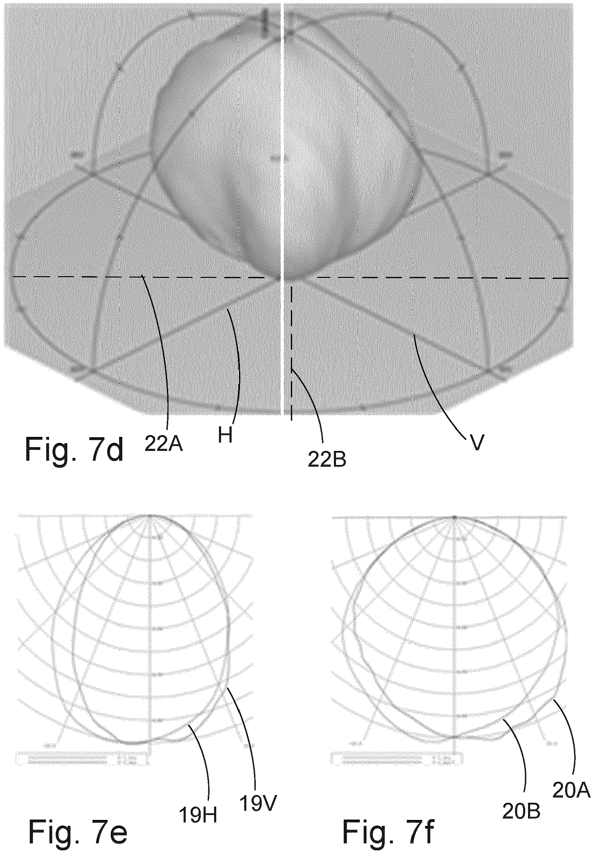

Fig. 7d illustrates luminous intensity distribution for the fifth embodiment in a 3-dimensional plot,
Fig. 7e illustrates luminous intensity distribution in the horizontal and the vertical plane for the fifth embodiment,
Fig. 7f illustrates luminous intensity distribution in the two diagonal planes for the fifth embodiment,

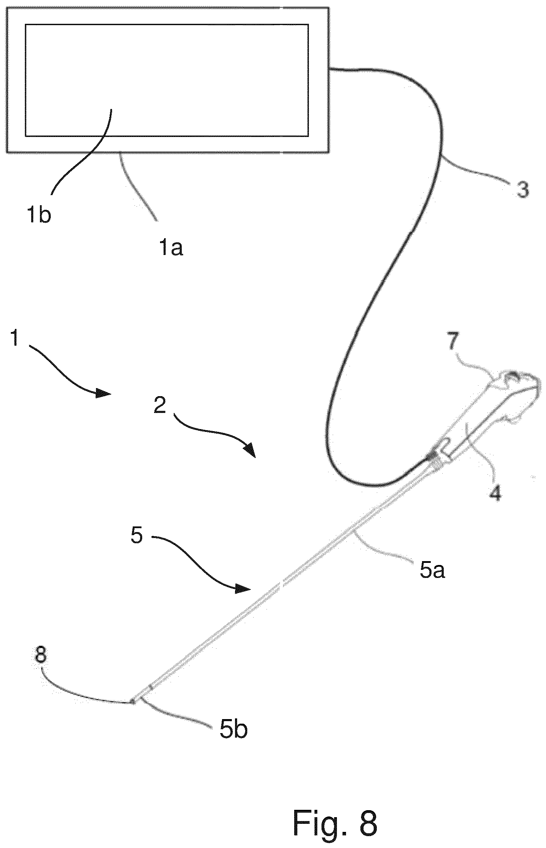

Fig. 8 shows a visualization system including an embodiment of a video processing apparatus and an endoscope with a tip part according to the disclosure,

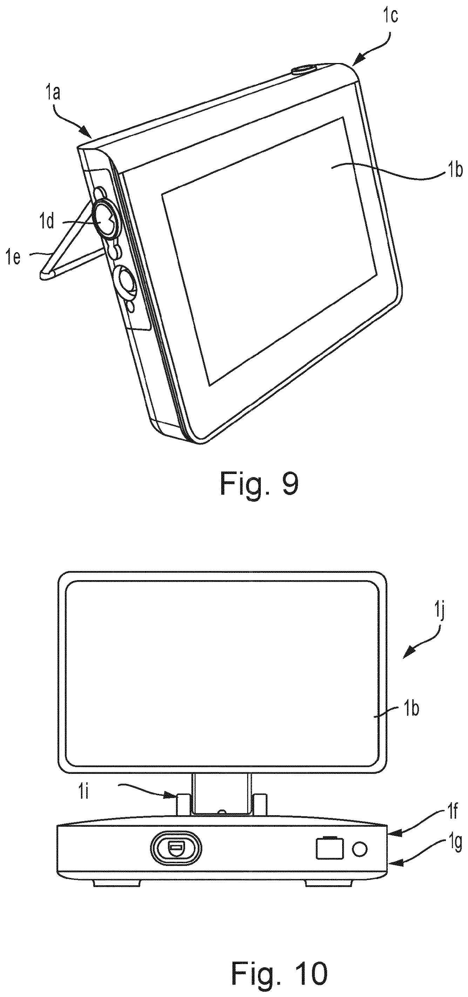

Fig. 9 is a perspective view of the video processing apparatus of Fig. 8,
Fig. 10 is a front view of another embodiment of the processing apparatus of Fig. 8,

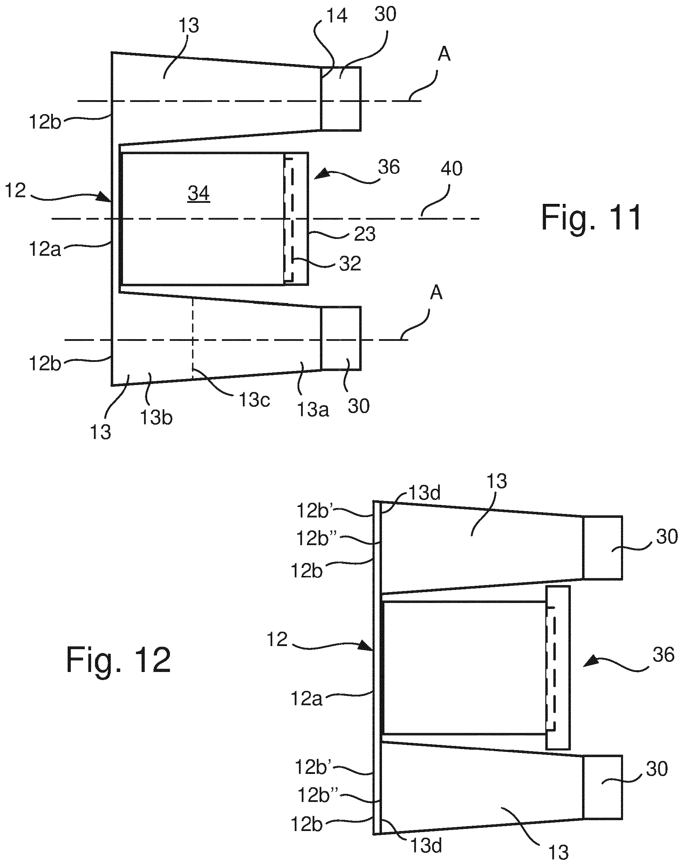

Fig. 11 is a block diagram of an embodiment of components of the tip part including the lightguide, and
Fig. 12 is a block diagram of a variation of the embodiment of Fig. 11.

### Detailed Description

Turning first to Fig. 8, an embodiment of a visualization system 1 according to the second aspect of the disclosure is shown. The visualization system 1 comprises a video processing apparatus (VPA), illustratively a VPA 1a (shown in block form) including a display screen 1b, and an endoscope 2 connectable thereto via a wireless connection, for example via a wireless HDMI protocol, or, as shown, a cable 3. The visualization system 1 is configured to display live images or video based on video obtained by an image sensor of the endoscope 2 along with a graphical user interface to control video or image presentation and capture functions.

The endoscope 2 is preferably a forward looking, disposable, i.e. single use, endoscope that is to be discarded after use in a patient, rather than cleaned, sterilized and reused. The endoscope 2 comprises a position interface, illustratively a handle 4, at the proximal end. The handle 4 is adapted to be gripped by the hand of an operator. The endoscope 2 also includes a bendable insertion cord 5 extending towards the distal end of the endoscope 2 and adapted to be inserted into a patient. The insertion cord 5 includes an insertion tube 5a, a bending section 5b, and a tip part 8 according to the disclosure connected to the bending section 5b. The bending section 5b is highly bendable. The bending motion of the bending section is controlled by the user using an operating member 7 such as knob or a lever via control cables (not visible) connected to the tip part 8 or the distal end of the bending section 5b. The bending section 5b comprises a thin covering sheath, and the inner details are thus not visible in Fig. 8.

Fig. 9 is a perspective view of the VPA 1a, which can be referred to as a monitor. The VPA 1a includes a housing 1c, a receptacle 1d to receive a connector of the cable 3, and a handle 1e that also functions as a table stand when the monitor is positioned on a table or other support structure. The housing 1c encloses the display screen 1b and circuitry operable to configure the live video captured by the image sensor to the requirements of the display screen 1b. The circuitry also generates a graphical user interface configured to enable the operator to control the image or video capture and presentation functions, as is known in the art.

Another embodiment of a VPA, illustratively VPA 1f, is shown in FIG. 10, together with a display device 1j including the display screen 1b and the receptacle 1d (not shown). The VPA 1a and the VPA 1f may perform the same video processing functions. The VPA 1f includes a housing and a display support interface 1i, which supports the display device 1j via a support arm. The display device 1j can be separated or removed from the VPA 1f. The processed video can also be presented with a remote or separate display device communicatively connected, via a wired or wireless connection, with the VPA 1f. This enables placement of the VPA 1f in a location distinct from the location of the display device. This also enables use of a display device available in the operating room for other purposes.

A position interface functions to control the position of the insertion cord 5. A handle is an example of a position interface and, unless stated otherwise, the terms are used interchangeably. The handle also functions to provide manual control actuators, e.g. knobs, levers, buttons, and the like, to steer the tip part and control instruments guided through the insertion cord. Alternatively, a different position interface can be provided that is connected to the insertion cord and is detachably connected to a robotic arm. The insertion cord thus extends from the robotic arm, and the endoscope is thus detachable from the robotic arm. The tip part (described below) is the same regardless of the position interface used. The robotic arm responds to signals, such as voice commands from the operator, to rotate, translate, and otherwise position the proximal end of the insertion cord, as an operator would do manually. The position interface can include control actuators, including manual control actuators. Alternatively or additionally, control actuators can be provided in or on the robotic arm or by the robotic system including the robotic arm, thereby potentially reducing the cost of the endoscope. Example control actuators include single axis actuators, including linear motion actuators. A linear motion actuator may comprise a threaded rod coupled to a threaded nut portion, in which a motor rotates the rod to translate the nut portion.

Turning now to Fig. 1, the housing 9 of the tip part 8 of the endoscope 2 is shown in greater detail. The housing 9 may be moulded integrally in a two-stage two component injection moulding process so as to have a transparent housing part 10 and an opaque housing part 11. The transparent housing part 10 comprises a front window part 12 preferably covering, as seen from the distal end, an electronic imaging device 23 (an example is shown in Figs. 11 and 12) provided in the tip housing 9. The front window part 12 furthermore comprises integrally formed lightguides 13 (shown in Fig. 11) adapted to guide and distribute light from light sources, such as LEDs 30 (shown in Figs. 11 and 12), also provided in the tip housing 9, in order to provide illumination for the imaging device. It is, however, not excluded that the electronic image capture device 23 is provided with its own front window (which may be shaped to function as a lens) and the lightguides 13 themselves constitute a front window part 12 or, if the electronic image capture device 23 is provided with its own front window, that the front window part 12 is excluded and the base of the lightguide (described below) provides a front-facing surface exposed to the environment. Although shown proximally of the electronic image capture device 23, the LEDs 30 can be placed distally of the electronic image capture device 23. Furthermore, the LEDs 30 can be adhesively bonded to the lightguides 13 or, alternatively, a support frame can be placed therebetween to maintain the lightguides or the LEDs or both in place relative to the image capture device 23.

The tip housing may also enclose a lens and a lens holder or barrel 34 (shown in Figs. 11 and 12). The electronic image capture device 23 has an optical axis 40. The cross-section of the image receptor 32 may be substantially rectangular or square, the shape defining a vertical and a horizontal imaging direction. The shape of the electronic image capture device 23 may also be circular or have other shapes. Typically, however, the image receptor 32, which converts light to pixel data constituting the live images, is substantially rectangular or square. It should be understood that a square is a species of a rectangle. The electronic image capture device 23 together with the lens(es) and associated circuitry (e.g. processor), may be referred to as a camera 36. The camera may be a commercially available wafer-level camera module communicatively coupled to the VPA by means of power connectors and serial communication connectors. The serial communication connectors establish serial communication via a serial communication protocol such as, for example, I2C. The VPA may provide configuration information to the camera on a periodic basis based on analysis, by the VPA, of the images to, for example, prevent overexposure or underexposure of the images. Such configuration information may comprise, for example, electronic shutter speed or exposure time.

The front window part 12 comprises a portion 12a (shown in Figs. 11 and 12) positioned across the optical axis 40 of the camera, which thus may be referred to as the camera window. The front window part 12 also comprises a portion or portions positioned distally from the light source or sources, which may be referred to as the light window(s) 12b (shown in Figs. 11 and 12). The camera window 12a and the light windows 12b can be integrally formed in one piece or can be provided as distinct pieces, as discussed above. Accordingly, the camera window can be combined and be part of the camera. A light window can be combined with and be part of a lightguide. Preferably, to reduce costs and complexity, the front window part 12 comprises, in one piece, the lightguides. The lightguides each comprise a proximal end 13a and a distal end 13b (shown in Fig. 11) and a circumferential surface surrounding a centre axis A and extending between said proximal end and said distal end. The circumferential surface is configured to provide internal reflection of light emitted from the light source and defines cross-sections 13c (an example shown in Fig. 11) of the lightguide, across the centre axis, the cross-sections comprising four corners arranged around the centre axis so as to define four edges subdividing the circumferential surface into four side surfaces. Each of the side surfaces comprises a curvature having an angle of curvature, the angle of curvature of each of the side surfaces increasing from the edges towards a middle of each of the side surfaces. Thus, the side surfaces, and the corresponding sides of the cross-sections, are curved.

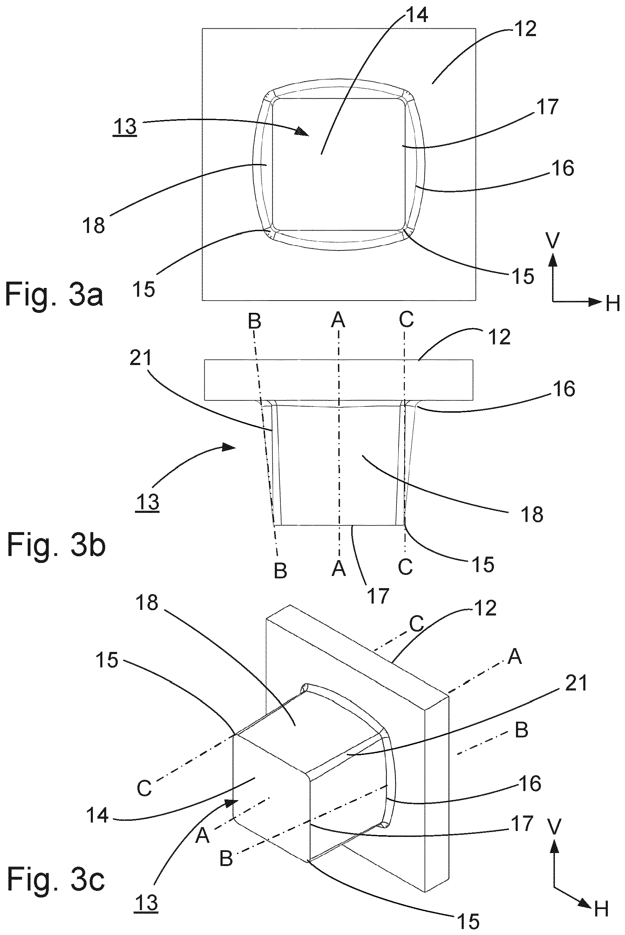

As can be seen, the lightguides 13 shown in Figs. 1 and 2 correspond specifically to the fifth embodiment shown in Figs. 7a-7c but may in other embodiments of the housing 9 be substituted with other lightguides 13, such as corresponding to those depicted in Figs. 3 to 6, as they all improve the light distribution according to the disclosure.

Turning now to Figs. 3a to 3c, a first embodiment of a lightguide 13 is shown as seen from the proximal end corresponding to the direction of Fig. 2. For simplicity, the front window part 12 is just schematically shown as a square but could of course have the full complexity of the front window part 12 of Figs. 1 and 2. The front window part 12 may be integrally moulded with the lightguide 13 but could also be a separate item. Furthermore, the end face of the lightguide 13 could itself constitute the front window. The same applies to other embodiments described below.

As can be seen, the body of the lightguide 13 is roughly the shape of a frustum of a four-sided pyramid. The base is contiguous with the front window part 12 and the top, i.e. the proximal end, comprises a plane surface 14 adapted to receive and be in good contact with the emission side of the LED. The plane surface 14 at the top is generally rectangular with four straight sides 17 except at the corners 15, which are slightly rounded for manufacturing reasons. Being a square, the corners are spaced with 90 degree spacing about a centre axis A-A. The sides are aligned with the horizontal imaging direction H and vertical imaging direction V defined by the image sensor or receptor of the electronic image capture device 23, schematically indicated with broken lines in Figs. 5a, 6a and 7a and the corresponding rectangular display on the display device. In the illustrated examples the image sensor 32 is assumed to be square meaning that the width w and the height h, as indicated on Fig. 6a, are equal, i.e. that the aspect ratio h/w is 1. However, as will be explained below, other aspect ratios are also possible, within the scope of invention. The cross-section at the base is not entirely rectangular but rather somewhat cushion-shaped with four convexly curved sides 16. As far as the lightguide 13 itself is considered, it ends at the base with the sides 16 because the front window part 12 beyond that distal end of the lightguide does not contribute with any internal reflection (when the front window part 12 extends laterally from the base). In practice there will be some curvature in the transition between base formed by the sides 16 of the lightguide 13 and the front window part 12, which is also not considered to be a part of the lightguide. The same applies to all other embodiments shown in the figures.

The four side surfaces 18 which together with the rounded corners form the circumferential surface of the lightguide 13 are each smooth surfaces without discontinuities with a curvature that (as seen from the outside) increases in convexity from the top towards the base. The four edges 21 formed by the corners are preferably perpendicular to the exit surface of the front window part 12, as indicated by the axis C-C, or as close to perpendicular as the manufacturing methods allow. That is ideally to say approximately 0° angle to the centre axis A-A about which the lightguide 13 exhibits a fourth order of symmetry. Some deviation may be acceptable for manufacturing reasons and the angle may thus be anywhere between 0° and 3°, preferably approximately 1 degree.

From the corner, when moving towards the middle of the side surface, the angle of the surfaces increase slightly from approximately 0° to a maximum angle of the side surfaces with respect to the centre axis A-A as indicated by the axis B-B. Because the maximum angle increases away from each corner the maximum angle is found midway on the surface 18 between two edges 21 defined by the corners. This maximum angle is preferably in the interval from 2° to 10°, but is always larger than the angle of the corners with respect to the centre line A-A.

With properly selected transparent materials this shape of the lightguide provides total internal reflection of the light from the light source, but because of the curvature of the side surfaces more light is emitted in the directions away from the corners of the lightguide 13. That is to say, the light is collimated more at the sides where the angles are higher than in the corners where the angles are lower. Suitable transparent materials could include Polycarbonate, Cyclic Olefin Polymer, Cyclic Olefin Copolymer, Styrene-Butadiene Copolymer, Silicone or Polystyrene. If the orientation of the edges 17 of the square shape of the plane surface 14 is aligned with the sides of the normally rectangular image sensors of the imaging device, more light will be available in the corners of the field of view. This can be seen from the example shown in Figs. 3d to Fig. 3f, where the curves 20A, 20B indicate that more light is emitted in the direction corresponding to the dashed axes 22A, 22B than in the direction corresponding to the fully drawn axes **H,** V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

Turning now to Fig. 4a to 4c, a second embodiment of a lightguide 13 is shown as seen from the proximal end corresponding to the direction of Fig. 2. Again, for simplicity, the front window part 12 is just schematically shown as a square but could of course have the full complexity of the front window part 12 of Figs. 1 and 2.

As can be seen, the body of the lightguide 13 is roughly the shape of a frustum of a four-sided pyramid. The base is preferably contiguous with the front window part 12 and the top, i.e. the proximal end, comprises a plane surface 14 adapted to receive and be in good contact with the emission side of a LED component. In this and other embodiments, the base could, however, also constitute the front window, or the front window could be a separate part. The plane surface 14 at the top is generally rectangular, however with four concavely curved edges 17, meeting at the corners 15, which are also in this embodiment slightly rounded for manufacturing reasons. The corners are spaced with 90 degree spacing about a centre axis A-A, so as to provide a fourth order of rotational symmetry. In this second embodiment the cross-section at the base is square with straight sides 16 and rounded corners. The sides are aligned with the horizontal imaging direction H and vertical imaging direction V defined by the image sensor and the corresponding rectangular display on the display device.

The four side surfaces 18 which together with the rounded corners form the circumferential surface of the lightguide 13 are each smooth surfaces without discontinuities with a curvature that, like the first embodiment, increases in convexity from the top towards the base or, depending on how you express it, decreases in concavity. Like the first embodiment, the four edges 21 formed by the corners are preferably perpendicular to the exit surface of the front window part 12, as indicated by the axis C-C, or as close to perpendicular as the manufacturing methods allow. That is ideally to say approximately 0° angle to the centre axis A-A about which the lightguide 13 exhibits a fourth order of symmetry. Some deviation may be acceptable for manufacturing reasons and the angle may thus be anywhere between 0° and 3°, preferably approximately 1 degree.

From the corner, the angle of the surfaces increases slightly from approximately 0° to a maximum angle of the side surfaces with respect to the centre axis A-A. Because the maximum angle increases away from each corner the maximum angle is found midway on the surface 18 between two edges 21 defined by the corners as indicated by the axis B-B. This maximum angle is preferably in the interval from 2° to 10°, but is always larger than the angle of the corners with respect to the centre line A-A.

With properly selected transparent materials this shape of the lightguide provides total internal reflection of the light from the light source, but because of the curvature of the side surfaces, the light is collimated more at the sides where the angles are higher than in the corners where the angles are lower, and accordingly light is emitted more to the sides than forwardly at the corners of the lightguide 13 than at the side surfaces where the angles are larger. If the orientation of the edges 17 of the plane surface 14 is aligned with the sides of the normally rectangular image sensors of the imaging device, more light will be available in the corners of the field of view. This can be seen from the example shown in Figs. 4d to Fig. 4f, where the curves 20A, 20B indicate that more light is emitted in the direction corresponding to the dashed axes 22A, 22B than in the direction corresponding to the fully drawn axes **H,** V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

Turning now to Figs. 5a to 5c an alternative embodiment of the lightguide 13 adapted for increasing light emission towards the corners of the field of view of the imaging device is shown. The lightguide 13 in this embodiment is not a truncated pyramid but rather a column. That is to say the base and the top 14 have essentially the same shape and area. Accordingly, the sides 18 and the edges 21 are perpendicular to the end surface, except for whatever deviation may be necessary for other reasons, i.e. small deviation in the interval from 0° to 3°. Such reasons may include manufacturing reasons but also collimation, e.g. to generally keep as much light as possible withing the field of view of the imaging device.

As can be seen the areas of top 14 and base has four corners 15 but is not quite square, but rather somewhat cushion-shaped, i.e. with a convex curvature as seen from the outside. This again applies to the entire surface outer 18 between the corners.

However, as seen from the inside of the lightguide 13 where incident light from the LED is subject to internal reflection the light is thus reflected of a concave surface. That is to say, the surface as seen from the inner of the light guide 13 is a concave mirror. Since, as will be understood, reflection is the issue, all references to convex and concave corners and surfaces will be made accordingly, i.e. as seen from the inside of the light guide 13, rather than in the conventional way for polygons in geometry. In this respect, corners will be understood as convex if the angle at which the surface meet, is larger than 180°, as seen from the inside, and concave if the angle with which they meet is smaller than 180°, as seen from the inside. In other words a convex corner points towards the centre of the light guide 13, and concave corners point away from the centre of the light guide 13. With the corners 15 arranged as two pairs of opposing concave corners 15 one pair opposing each other in the vertical direction V and the other pair opposing each other in the horizontal direction H, as defined with respect to the image receptor and display, it is possible to reflect more light towards the corners of the image than the sides of the field of view of the square image receptor as illustrated in Fig 5a. As can be seen the curvature of the surface 18 is concave and so selected that light impinging midway on the surface is reflected straight back towards the centre line A-A whereas light impinging closer to and at the corners 15 are reflected back at an angle so that all light is generally directed more in a direction between the horizontal and vertical directions. The curvature may be selected differently from that shown, but ideally at the corners the angle β of the tangent to the surface with respect to vertical or horizontal direction should be 22.5° or at least in the interval between 20° and 25° in order to achieve the desired reflection at an angle of 45° with respect to vertical or horizontal as illustrated. In the example illustrated in Figs. 5a to 5c, this results in a concave corner 15 as seen from within the lightguide 13 with an angle α of 135° or at least between 125° and 145°.

Thus, with opposing corners 15 and resulting edges 12 arranged along the vertical and horizontal direction and suitably selected angles of the corners 15, more light will be available in the corners of the field of view of the imaging device. This can be seen from the example shown in Figs. 5d to Fig. 5f, where the curves 20A, 20B indicate that more light is emitted in the direction corresponding to the dashed axes 22A, 22B than in the direction corresponding to the fully drawn axes **H,** V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture.

Irrespective of whether the corners are arranged exactly opposite each other and whether the image sensor is generally rectangular i.e. height h differs from width w, the curvature between two neighbouring corners is so selected that the reflection angle Θ with respect to horizontal H is so selected that the reflection angle (Θ) is either ± arctan (h/w), or ±(180° - arctan (h/w)). That is to say if the aspect ratio of the image sensor is not 1, the angles towards the corner are not 45°, the curvature is still adapted to reflect light towards the corners, and does thus not deviate from the scope of the disclosure.

As illustrated in Fig. 5a this can be achieved if the tangent T to the reflecting surface is at an angle Θ₂ to horizontal, where Θ₂ is so selected that Θ = 2Θ₂ - Θ₁, where Θ₁ is the emission angle of a light ray emanating from the centre axis with respect to said horizontal direction H.

In Figs. 6a to 6c another embodiment of the lightguide 13 adapted for increasing light emission towards the corners of the field of view of the imaging device is shown. Like the lightguide illustrated in Figs. 5a-5c, the lightguide 13 in this embodiment is also not a truncated pyramid but rather a column. That is again to say the base and the top 14 have essentially the same shape and area. Accordingly, the sides 18 and the edges 21 are perpendicular to the end surface, except for whatever deviation may be necessary for other reasons, i.e. small deviation in the interval from 0° to 3°. Such reasons may include manufacturing reasons but also collimation, e.g. to generally keep as much light as possible withing the field of view of the imaging device. As can be seen the areas of top 14 is also not quite square, and has not only four corners 15 arranged as two pairs of opposing corners 15 one pair opposing in the vertical direction V and the horizontal direction H, as defined with respect to the image receptor and display, but four additional corners 15'. These additional corners are essential concave right-angled corners as seen from the inside of the lightguide 13.

As will be noticed, the opposing corners 15 in the vertical direction and the horizontal direction are not concave corners 15 but instead convex corners 15.

As seen from the inside of the lightguide 13 incident light from the LED is subject to internal reflection the light is also in this embodiment reflected off a concave surface 18, of which there are eight. The curvature of the surface 18 is so selected that no light impinging on any of the surfaces 18 is reflected straight back towards the centre line A-A but all reflected back at an angle so that all light is generally directed more in a direction between the horizontal and vertical directions.

The curvature may be selected differently from that shown, but ideally at the corners the angle β of the tangent to the surface with respect to vertical or horizontal direction should be 22.5° (or -22.5°) in order to achieve the desired reflection at an angle of 45 ° with respect to vertical or horizontal as illustrated for the square image receptor 23. In the example illustrated in Figs. 6a to 6c, this results in a convex corner 15 as seen from within the lightguide 13 with an angle α of 225°.

Thus, with opposing corners 15 and resulting edges 12 arranged along the vertical and horizontal direction and suitably selected angles of the corners 15, more light will be available in the corners of the field of view of the imaging device. This can be seen from the example shown in Figs. 6d to Fig. 6f, where the curves 20A, 20B indicate that more light is emitted in the direction corresponding to the dashed axes 22A, 22B than in the direction corresponding to the fully drawn axes **H,** V corresponding to the curves 19H, 19V. Deviations may occur as the emission from the LEDs is not necessarily coincident with the centre of the emission surface of the LED component, in turn involving a potential misalignment with the centre of the lightguide 13 that needs to be minimized during manufacture. It will be noticed that the light emission towards the corners of the field of view is better for this embodiment than for the previously described embodiment, but that this comes at the expense of higher complexity, such as *inter alia* the sharp corners 15', leading to more complicated tooling in manufacture.

Similar to the previously described embodiment, irrespective of whether the corners are arranged exactly opposite each other, the curvature between two neighbouring corners is so selected that the reflection angle Θ with respect to horizontal H is so selected that the reflection angle (Θ) is either ± arctan (h/w), or ±(180° - arctan (h/w)). That is to say, if the aspect ratio of the image sensor 23 differs from 1 and the angles towards the corner are not 45°, the curvature is still adapted to reflect light towards the corners. As can be seen from Fig. 6a, this again can be achieved when the tangent T to the reflecting surface is at an angle Θ₂ to horizontal, where Θ₂ is so selected that Θ = 2Θ₂ - Θ₁, where Θ₁ is the emission angle of a light ray emanating from the centre axis with respect to said horizontal direction H.

A further embodiment of the lightguide 13, which can be seen as a hybrid between the two previously described embodiments is shown in Figs. 7a to 7c avoids these sharp corners. This embodiment, which as the two previously described embodiments, comprises only concave inner surfaces 18 for total internal reflection, but has another four additional corners 15' compared to figs. 6a to 6c and accordingly twelve corners 15, 15' in total and a corresponding number of concave surfaces 18 for total internal reflection. The shown embodiment is, like the previous ones especially adapted for a square image receptor 23, but is like those previous embodiments, also generally applicable to other aspect ratios without deviating from the disclosure.

It thus has not only four convex corners 15, arranged as two pairs of opposing corners 15, one pair opposing in the vertical direction V and the horizontal direction H, as defined with respect to the image receptor and display, but eight additional corners 15'. These additional corners 15' are essential concave corners as seen from the inside of the lightguide 13, but with a much more obtuse angle than in the previously described embodiment. The additional surface 18 provided this way provides internal reflections similar to the ones achieved in the embodiment of Figs. 5a to Fig. 5c, i.e. more backwards in direction of the centre line A-A from which it was ideally emitted.

Accordingly, the curvature between neighbouring corners is so selected that the reflection angle Θ with respect to horizontal H is so selected that the selected that the reflection angle (Θ) is either ± arctan (h/w), or ±(180° - arctan (h/w)). So also here, if the aspect ratio of the image sensor 23 differs from 1 and the angles towards the corner are not 45°, the curvature is still adapted to reflect light towards the corners.. Although not shown, this is also in this case fulfilled when the tangent T to the reflecting surface is at an angle Θ₂ to horizontal, where Θ₂ is so selected that Θ = 2Θ₂ - Θ₁, where Θ₁ is the emission angle of a light ray emanating from the centre axis with respect to said horizontal direction H.

Further corners 15', i.e. higher multiples of four than eight can be envisaged.

The curvature may be selected differently from that shown, but ideally at the convex corners 15 as seen from within the lightguide 13 are also with an angle of 225° in this embodiment.

In general, for the above embodiments, especially adapted for image sensors with square imaging surface, it should be noted that arctan (1/1) is 45. Accordingly, the reflection angle may also be expressed as: Θ = -45° + N · 90°, where N is an integer.

As mentioned above, if the aspect ratio differs from 1, as in many commonly used rectangular image sensor formats, the angles towards the corners will differ.

Thus if h/w = 9/16, the angles Θ will be ± arctan (9/16), or ±(180° - arctan (9/16)) or approximately: 29.4°=(arctan(9/16)), 150.6°=(180°-arctan(9/16)), 209.4° (-150.6°) or 330.6° (-29.4°).

Similarly, if h/w = 3/4, the angles Θ will be ± arctan (3/4), or ±(180° - arctan (3/4)) or approximately: 36.9°=(arctan(3/4)), 143.1°=(180°-arctan(3/4)), 216.9° (-143.1°) or 323.1° (-36.9).

It may furthermore be envisaged that features of the embodiments of Fig. 3 and Fig. 4 may be combined with the features of the embodiments of Figs. 5 to Fig. 7 and vice versa.

Turning now to Figs. 11 and 12, there are shown two variations of components of the tip part discussed above, including the front window part 12 preferably covering, as seen from the distal end, the electronic imaging device 23. As seen in Fig. 11, the front window part 12 includes a camera window 12a and two light windows 12b. Integrally formed therewith are the lightguides 13, including proximal ends 13a, distal ends 13b, an arbitrarily placed line 13c denoting a cross-section of the lightguide 13, light sources 30 adjacent the plane surfaces 14 of the light guides, and axis A of the lightguides. In Figs. 11 and 12 the lightguides are shown without the curvature of the side surfaces, for simplicity. In Fig. 12 the camera window 12a and the light windows 12b are not integrally formed with the lightguides 13, thus are shown the base or distal face 13d of the lightguides 13 and the abutting surfaces 12b" of the light windows, opposite the surfaces 12b' of the light windows, which face the environment. As discussed, in some variations the light windows are integrally formed with the lightguides but not the camera window, which could be incorporated with the electronic image capture device. In other variations the light windows are omitted and the distal face 13d of the lightguides face the environment.

Although shown proximally of the electronic image capture device 23, the LEDs 30 can be placed distally of the electronic image capture device 23. Furthermore, the LEDs 30 can be adhesively bonded to the lightguides 13 or, alternatively, a support frame can be placed therebetween to maintain the lightguides or the LEDs or both in place relative to the image capture device 23.

The electronic image capture device 23 has an optical axis 40. The cross-section of the image receptor 32 may be substantially rectangular or square. The electronic image capture device 23 together with the lens(es), lens holder 34, and associated circuitry (e.g. image receptor and processor included in an integrated circuit of the electronic image capture device 23), may be referred to as a camera 36. The camera 36 may be a commercially available wafer-level camera module. The camera may be communicatively coupled to the VPA by means of power connectors and serial communication connectors. The serial communication connectors establish serial communication via a serial communication protocol such as, for example, I2C. The VPA may provide configuration information to the camera on a periodic basis based on analysis, by the VPA, of the images to, for example, prevent overexposure or underexposure of the images. Such configuration information may comprise, for example, electronic shutter speed or exposure time.

## Claims

1. An endoscope (2) comprising an insertion cord (5), the insertion cord comprising a distal tip (8), said distal tip being arranged at the distal end of the insertion cord, said distal tip comprising
an electronic image capture device (23) having an image receptor (32) having a vertical image direction (V) with a height (h) and a horizontal (H) imaging direction with a width (w),
a light source (30),
a lightguide (13) positioned distally from the light source, the lightguide comprising a proximal end (13a), a distal end (13b) and a circumferential surface surrounding a centre axis (A) and extending between said proximal end and said distal end,
wherein said circumferential surface is configured to provide internal reflection of light emitted from the light source, and said circumferential surface defines cross-sections of said lightguide, wherein said cross-sections comprise four corners (15) arranged around said centre axis, the four corners subdividing the circumferential surface into side surfaces (18),
wherein each of said side surfaces comprises a curvature having an angle of curvature,
**characterised in that**
said curvature is so selected that incident light emitted from said centre axis is reflected at an angle (Θ) with respect to said horizontal direction (H), where (Θ) is either ± arctan (h/w), or ±(180° - arctan (h/w)).

2. An endoscope according to claim 1, wherein the cross-section comprises four curved surfaces joining in four concave corners where the angles of the joining surfaces at the concave corners are approximately 135°.

3. An endoscope according to claim 1, wherein the cross-section comprises eight curved surfaces joining in four concave corners and four convex corners where the angles of the joining surfaces at the concave corners are approximately 90° and the angles of the joining surfaces at the convex corners are approximately 225°.

4. An endoscope according to claim 1, wherein the cross-section comprises twelve curved surfaces joining in eight concave corners and four convex corners where the angles of the joining surfaces at the concave corners are approximately 135° and the angles of the joining surfaces at the concave corners are approximately 225°.

5. An endoscope according to claim 1, wherein the curvature of each of said side surfaces is the same.

6. An endoscope according to claim 1, wherein each of said corners extends between the proximal end and the distal end at an angle with respect to the centre axis.

7. An endoscope according to claim 6, wherein the angle of the corners with respect to the centre axis is between 0 and 3 degrees, preferably approximately 1 degree.

8. An endoscope according to claim 1, wherein a maximum angle of the side surfaces with respect to the centre axis is in the interval from 2 to 10, preferably approximately 5 degrees.

9. An endoscope according to claim 1, wherein said curvature is given by the formula: Θ = 2Θ₂ - Θ₁, where Θ₁ is the emission angle of a light ray with respect to said horizontal direction and Θ₂ is the angle of the tangent to the circumferential surface with respect to horizontal at the point of incidence of said light ray.

10. An endoscope according to claim 9, wherein said cross-section defines four corners arranged with a 90° spacing around said centre axis so as to provide a first pair and a second pair of mutually opposing corners, where a first corner of the first pair opposes the second corner of the first pair along a first diagonal coincident with said vertical imaging direction (V), and
where a first corner of the second pair opposes the second corner of the second pair along a second diagonal coincident with said horizontal (H) imaging direction.

11. An endoscope according to claim 10, wherein further corners are provided between said first pair of mutually opposing corners and said second pair of mutually opposing corners.

12. An endoscope according to claim 1, wherein said cross-section defines four corners arranged with a 90° spacing around said centre axis so as to provide a first pair and a second pair of mutually opposing corners, where a first corner of the first pair opposes the second corner of the first pair along a first diagonal coincident with said vertical imaging direction (V), and
where a first corner of the second pair opposes the second corner of the second pair along a second diagonal coincident with said horizontal (H) imaging direction.

13. An endoscope according to claim 12, wherein further corners are provided between said first pair of mutually opposing corners and said second pair of mutually opposing corners.

14. An endoscope according to any one of claims 9 to 13, wherein the number of further corners is a multiple of four, in particular four or eight.

15. A visualization system (1) comprising a video processing apparatus (VPA) (1a) and an endoscope (2) according to any one of the preceding claims. wherein the endoscope is connectable to said VPA.

## Patentansprüche

1. Endoskop (2), umfassend ein Einführkabel (5), wobei das Einführkabel eine distale Spitze (8) umfasst, wobei die distale Spitze an dem distalen Ende des Einführkabels angeordnet ist, wobei die distale Spitze umfasst
eine elektronische Bildaufnahmevorrichtung (23), die einen Bildrezeptor (32) aufweist, der eine vertikale Bildrichtung (V) mit einer Höhe (h) und einer horizontalen (H) Bildaufnahmerichtung mit einer Breite (w) aufweist,
eine Lichtquelle (30),
eine Lichtführung (13),
die distal von der Lichtquelle positioniert ist, wobei die Lichtführung ein proximales Ende (13a), ein distales Ende (13b) und eine Umfangsfläche umfasst, die eine Mittelachse (A) umgibt und sich zwischen dem proximalen Ende und dem distalen Ende erstreckt,
wobei die Umfangsfläche dazu ausgelegt ist, interne Reflexion von Licht bereitzustellen, das von der Lichtquelle emittiert wurde, und die Umfangsfläche Querschnitte der Lichtführung definiert, wobei die Querschnitte vier Ecken (15) umfassen, die rund um die Mittelachse angeordnet sind, wobei die vier Ecken die Umfangsfläche in Seitenflächen (18) unterteilen,
wobei jede der Seitenflächen eine Krümmung mit einem Krümmungswinkel umfasst, **dadurch gekennzeichnet, dass**
die Krümmung derart ausgewählt ist, dass einfallendes Licht, das von der Mittelachse emittiert wird, in einem Winkel (Θ) mit Bezug auf die horizontale Richtung (H) reflektiert wird, wobei (Θ) entweder ± arctan (h/w) oder ±(180° - arctan (h/w)) ist.

2. Endoskop nach Anspruch 1, wobei der Querschnitt vier gekrümmte Flächen umfasst, die an vier konkav gewölbten Ecken verbunden sind, wobei die Winkel der Verbindungsflächen an den konkav gewölbten Ecken ungefähr 135° betragen.

3. Endoskop nach Anspruch 1, wobei der Querschnitt acht gekrümmte Flächen umfasst, die an vier konkav gewölbten Ecken und vier konvex gewölbten Ecken verbunden sind, wobei die Winkel der Verbindungsflächen an den konkav gewölbten Ecken ungefähr 90° betragen und die Winkel der Verbindungsflächen an den konvex gewölbten Ecken ungefähr 225° betragen.

4. Endoskop nach Anspruch 1, wobei der Querschnitt zwölf gekrümmte Flächen umfasst, die an acht konkav gewölbten Ecken und vier konvex gewölbten Ecken verbunden sind, wobei die Winkel der Verbindungsflächen an den konkav gewölbten Ecken ungefähr 135° betragen und die Winkel der Verbindungsflächen an den konkav gewölbten Ecken ungefähr 225° betragen.

5. Endoskop nach Anspruch 1, wobei die Krümmung einer jeden der Seitenflächen dieselbe ist.

6. Endoskop nach Anspruch 1, wobei sich jede der Ecken zwischen dem proximalen Ende und dem distalen Ende in einem Winkel mit Bezug auf die Mittelachse erstreckt.

7. Endoskop nach Anspruch 6, wobei der Winkel der Ecken mit Bezug auf die Mittelachse zwischen 0 und 3 Grad, vorzugsweise ungefähr 1 Grad beträgt.

8. Endoskop nach Anspruch 1, wobei ein maximaler Winkel der Seitenflächen mit Bezug auf die Mittelachse im Intervall von 2 bis 10 liegt, vorzugsweise ungefähr 5 Grad beträgt.

9. Endoskop nach Anspruch 1, wobei die Krümmung berechnet wird mit der Formel: Θ = 2Θ₂ - Θ₁, worin Θ₁ der Emissionswinkel eines Lichtstrahls mit Bezug auf die horizontale Richtung und Θ₂ der Winkel der Tangente zur Umfangsfläche mit Bezug auf die Horizontale am Einfallspunkt des Lichtstrahls ist.

10. Endoskop nach Anspruch 9, wobei der Querschnitt vier Ecken definiert, die mit einem 90°-Abstand rund um die Mittelachse angeordnet sind, um ein erstes Paar und ein zweites Paar einander gegenüberliegender Ecken bereitzustellen, wobei eine erste Ecke des ersten Paares der zweiten Ecke des ersten Paares entlang einer ersten Diagonale gegenüberliegt, die mit der vertikalen Bildaufnahmerichtung (V) zusammenfällt, und
wobei eine erste Ecke des zweiten Paares der zweiten Ecke des zweiten Paares entlang einer zweiten Diagonale gegenüberliegt, die mit der horizontalen (H) Bildaufnahmerichtung zusammenfällt.

11. Endoskop nach Anspruch 10, wobei weitere Ecken zwischen dem ersten Paar einander gegenüberliegender Ecken und dem zweiten Paar einander gegenüberliegender Ecken bereitgestellt sind.

12. Endoskop nach Anspruch 1, wobei der Querschnitt vier Ecken definiert, die mit einem 90°-Abstand rund um die Mittelachse angeordnet sind, um ein erstes Paar und ein zweites Paar einander gegenüberliegender Ecken bereitzustellen, wobei eine erste Ecke des ersten Paares der zweiten Ecke des ersten Paares entlang einer ersten Diagonale gegenüberliegt, die mit der vertikalen Bildaufnahmerichtung (V) zusammenfällt, und
wobei eine erste Ecke des zweiten Paares der zweiten Ecke des zweiten Paares entlang einer zweiten Diagonale gegenüberliegt, die mit der horizontalen (H) Bildaufnahmerichtung zusammenfällt.

13. Endoskop nach Anspruch 12, wobei weitere Ecken zwischen dem ersten Paar einander gegenüberliegender Ecken und dem zweiten Paar einander gegenüberliegender Ecken bereitgestellt sind.

14. Endoskop nach einem der Ansprüche 9 bis 13, wobei die Anzahl weiterer Ecken ein Vielfaches von vier, insbesondere vier oder acht ist.

15. Visualisierungssystem (1), umfassend eine Videoverarbeitungsvorrichtung (VPA) (1a) und ein Endoskop (2) nach einem der vorhergehenden Ansprüche, wobei das Endoskop mit der VPA verbindbar ist.

## Revendications

1. Endoscope (2) comprenant un cordon d'insertion (5), le cordon d'insertion comprenant une pointe distale (8), ladite pointe distale étant agencée à l'extrémité distale du cordon d'insertion, ladite pointe distale comprenant un dispositif de capture d'image électronique (23) comportant un récepteur d'image (32) ayant une direction d'image verticale (V) avec une hauteur (h) et une direction d'imagerie horizontale (H) avec une largeur (w),
une source lumineuse (30),
un guide de lumière (13)
positionné distalement par rapport à la source lumineuse, le guide de lumière comprenant une extrémité proximale (13a), une extrémité distale (13b) et une surface circonférentielle entourant un axe central (A) et s'étendant entre ladite extrémité proximale et ladite extrémité distale,
ladite surface circonférentielle étant configurée pour fournir une réflexion interne de la lumière émise par la source lumineuse, et ladite surface circonférentielle définissant des sections transversales dudit guide de lumière, lesdites sections transversales comprenant quatre coins (15) agencés autour dudit axe central, les quatre coins subdivisant la surface circonférentielle en surfaces latérales (18),
chacune desdites surfaces latérales comprenant une courbure ayant un angle de courbure, **caractérisé en ce que**
ladite courbure est choisie de telle sorte que la lumière incidente émise par ledit axe central soit réfléchie selon un angle (θ) par rapport à ladite direction horizontale (H), (θ) étant soit ± arctan (h/w), soit ±(180° - arctan (h/w)).

2. Endoscope selon la revendication 1, la section transversale comprenant quatre surfaces courbes se rejoignant dans quatre coins concaves, les angles des surfaces de jonction aux coins concaves étant approximativement de 135°.

3. Endoscope selon la revendication 1, la section transversale comprenant huit surfaces incurvées se rejoignant dans quatre coins concaves et quatre coins convexes, les angles des surfaces de jonction au niveau des coins concaves étant approximativement de 90° et les angles des surfaces de jonction au niveau des coins convexes étant approximativement de 225°.

4. Endoscope selon la revendication 1, la section transversale comprenant douze surfaces incurvées se rejoignant dans huit coins concaves et quatre coins convexes, les angles des surfaces de jonction aux coins concaves étant approximativement de 135° et les angles des surfaces de jonction aux coins concaves étant approximativement de 225°.

5. Endoscope selon la revendication 1, la courbure de chacune desdites surfaces latérales étant la même.

6. Endoscope selon la revendication 1, chacun desdits coins s'étendant entre l'extrémité proximale et l'extrémité distale selon un angle par rapport à l'axe central.

7. Endoscope selon la revendication 6, l'angle des coins par rapport à l'axe central étant compris entre 0 et 3 degrés, de préférence d'environ 1 degré.

8. Endoscope selon la revendication 1, un angle maximal des surfaces latérales par rapport à l'axe central étant compris dans l'intervalle de 2 à 10, de préférence d'environ 5 degrés.

9. Endoscope selon la revendication 1, ladite courbure étant donnée par la formule : θ = 2Θ₂ - Θ₁, Θ₁ étant l'angle d'émission d'un rayon lumineux par rapport à ladite direction horizontale et Θ₂ étant l'angle de la tangente à la surface circonférentielle par rapport à l'horizontale au point d'incidence dudit rayon lumineux.

10. Endoscope selon la revendication 9, ladite section transversale définissant quatre coins agencés avec un espacement de 90° autour dudit axe central de manière à fournir une première paire et une seconde paire de coins mutuellement opposés, un premier coin de la première paire étant opposé au second coin de la première paire le long d'une première diagonale coïncidant avec ladite direction d'imagerie verticale (V), et
un premier coin de la seconde paire étant opposé au second coin de la seconde paire le long d'une seconde diagonale coïncidant avec ladite direction d'imagerie horizontale (H).

11. Endoscope selon la revendication 10, des coins supplémentaires étant prévus entre ladite première paire de coins mutuellement opposés et ladite seconde paire de coins mutuellement opposés.

12. Endoscope selon la revendication 1, ladite section transversale définissant quatre coins agencés avec un espacement de 90° autour dudit axe central de manière à fournir une première paire et une seconde paire de coins mutuellement opposés, un premier coin de la première paire étant opposé au second coin de la première paire le long d'une première diagonale coïncidant avec ladite direction d'imagerie verticale (V), et
un premier coin de la seconde paire étant opposé au second coin de la seconde paire le long d'une seconde diagonale coïncidant avec ladite direction d'imagerie horizontale (H).

13. Endoscope selon la revendication 12, des coins supplémentaires étant prévus entre ladite première paire de coins mutuellement opposés et ladite seconde paire de coins mutuellement opposés.

14. Endoscope selon l'une quelconque des revendications 9 à 13, le nombre des coins supplémentaires étant un multiple de quatre, en particulier quatre ou huit.

15. Système de visualisation (1) comprenant un appareil de traitement vidéo (VPA) (1a) et un endoscope (2) selon l'une quelconque des revendications précédentes, l'endoscope pouvant être connecté audit VPA.
